(19) Europäisches Patentamt

European Patent Office

Office européen des brevets

(11) **EP 0 667 774 B1**

## (12) EUROPÄISCHE PATENTSCHRIFT

(45) Veröffentlichungstag und Bekanntmachung des Hinweises auf die Patenterteilung:
**06.08.1997 Patentblatt 1997/32**

(21) Anmeldenummer: **93923558.6**

(22) Anmeldetag: **27.10.1993**

(51) Int. Cl.$^6$: **A61K 31/40**, A61K 9/70, A61K 47/10

(86) Internationale Anmeldenummer:
**PCT/EP93/02970**

(87) Internationale Veröffentlichungsnummer:
**WO 94/10999 (26.05.1994 Gazette 1994/12)**

(54) **TRANSDERMALES THERAPEUTISCHES SYSTEM ZUR VERABREICHUNG VON PHYSOSTIGMIN AN DIE HAUT UND VERFAHREN ZU DESSEN HERSTELLUNG**

TRANSDERMAL THERAPEUTIC SYSTEM FOR ADMINISTERING PHYSOSTIGMIN THROUGH THE SKIN, AND A METHOD OF PRODUCING THE SYSTEM

SYSTEME THERAPEUTIQUE TRANSDERMIQUE POUR L'ADMINISTRATION DE PHYSOSTIGMINE SUR LA PEAU, ET SON PROCEDE DE FABRICATION

(84) Benannte Vertragsstaaten:
**AT BE CH DE DK ES FR GB GR IE IT LI LU MC NL PT SE**

(30) Priorität: **12.11.1992 DE 4238223**

(43) Veröffentlichungstag der Anmeldung:
**23.08.1995 Patentblatt 1995/34**

(73) Patentinhaber:
• **LTS LOHMANN Therapie-Systeme GmbH**
  **56567 Neuwied (DE)**
• **Klinge Pharma GmbH**
  **D-81673 München (DE)**

(72) Erfinder:
• **DEURER, Lothar**
  **D-56077 Koblenz (DE)**

• **HILLE, Thomas**
  **D-56564 Neuwied (DE)**
• **PROFITLICH, Thomas**
  **D-81547 München (DE)**
• **STANISLAUS, Fritz**
  **D-81673 München (DE)**
• **WALTER, Kersten**
  **D-80469 München (DE)**

(74) Vertreter: **Flaccus, Rolf-Dieter, Dr.**
**Patentanwalt**
**Sperlingsweg 32**
**50389 Wesseling (DE)**

(56) Entgegenhaltungen:
EP-A- 0 376 067          EP-A- 0 377 147
WO-A-91/12785          WO-A-91/15176
GB-A- 2 163 347          GB-A- 2 171 906

**Beschreibung**

Die Erfindung betrifft ein transdermales therapeutisches System, das Physostigmin als wirksamen Bestandteil enthält, sowie ein verfahren zu seiner Herstellung.

Die Applikation von Physostigmin, beispielsweise zur Behandlung der Alzheimer-Krankheit, wurde in der Literatur mehrfach beschrieben, wobei die Wirksamkeit der Substanz unterschiedlich beurteilt wird. Da das Alkaloid einen hohen first pass effect besitzt - die Bioverfügbarkeit des Physostigmins nach oraler Gabe liegt bei 5 % - sind die abweichenden Ergebnisse auf Varianten der Applikation zurückzuführen.

In der DE-OS 35 28 979 wird eine Zusammensetzung beschrieben, die neben Physostigmin eine Carbonsäure mittlerer Kettenlänge enthält; diese Zusammensetzung kann auf einer Bandage, Einlage oder Kompresse getragen werden, die mittels Verband aufgebracht wird. Bei dieser Applikationsart, die an sich kein TTS darstellt, ist vorgesehen, die Bandage, Kompresse oder Einlage mit einer Reservoir-Innenschicht, einer undurchlässigen Schutzsperrfolie oder einem undurchlässigen Schutzfilm zu versehen und zwischen Reservoir und der Haut eine Diffusionssteuermembran anzubringen. Weder die Diffusionssteuermembran, noch die Schutzfolien sind näher beschrieben. Die Carbonsäuren werden ausdrücklich als ein wirksames Transportvehikel für die Verabreichung des Arzneimittels durch die Haut bezeichnet, das sonst nicht durch die Hautbarriere hindurchtreten könnte. Diese Aussage ist jedoch wissenschaftlich nicht haltbar. In der DE-PS 36 06 892 wird eine retardierte Applikation von Phyosostigmin und anderen Wirkstoffen beschrieben, die transdermal erfolgen kann. Eine spezielle Formulierung wird nicht offenbart, vielmehr wird auf ein bereits beschriebene Formulierung nach der US-PS 3,921,363 verwiesen.

Eine weitere Offenlegungsschrift, die die transdermale Applikation von Physostigmin beschreibt, ist WO 91/15176. Diese Offenlegungsschrift geht nicht über die Lehre des Patents DE 38 43 239 hinaus, das weiter unten gewürdigt werden wird. Neben den nur vagen Ausführungen der transdermalen therapeutischen Systeme wird in keinem der vorstehend erwähnten Dokumente auf die Instabilität von Physostigmin eingegangen, die schon früh erkannt wurde. Hierzu kann jedoch beispielsweise auf die folgenden Literaturstellen hingewiesen werden, in welchen die Instabilität von Physostigmin behandelt wird:
(Eber, W., Pharmaz. Ztg. 37, 483 (1988), Herzig, J., Mayer, H., Mh. Chem. 18, 379 (1987); Herzig, J., Lieb, H., ebenda 39, 285 (1918); Solvay, A. A., J. chem. Soc. (London) 101, 978 (1912));

Diese Instabilität setzt aufgrund einer raschen Zersetzung des Wirkstoffes dem Einsatz des Physostigmins in der Pharmazie enge Grenzen.

Das Problem der raschen Zersetzung von Physostigmin in einem TTS ist weiterhin den Patentschriften DE 38 43 238 und DE 38 43 239 zu entnehmen. Das in DE 38 43 239 beschriebene TTS enthält ausschließlich lipophile Weichmacher, während das in DE 38 43 238 beschriebene System neben lipophilen Weichmachern höherkettige Carbonsäuren, nämlich Ölsäure oder Undecensäure, als Lösungsmittel für Physostigmin enthält. Diese Fettsäuren sind ebenfalls lipophile Stoffe. Daher ist beiden Systemen gemeinsam, daß Physostigmin aus einer lipophilen Matrix, die die Stabilität des Wirkstoffs garantiert, an die Haut abgegeben wird.

Überraschenderweise wurde durch Nachprüfungen festgestellt, daß TTS, die nach den Lehren der Patentschriften DE 38 43 238 und DE 38 43 239 hergestellt wurden, nicht den bei einem TTS an die Eigenklebrigkeit zu stellenden strengen Anforderungen in zufriedenstellender Weise entsprechen. Trageversuche hatten das Ergebnis, daß die TTS bereits nach achtstündigem Tragen nicht mehr bei allen Probanden vollflächig klebten. Nach 24 Stunden hatten 10 % der Probanden die TTS verloren, und bei 15 % der Probanden bestand kein vollflächicher Hautkontakt mehr. Lediglich bei 75 % der Probanden war das Klebeverhalten der TTS zufriedenstellend. TTS, die nur bei 75 % der Probanden bzw. Patienten ohne Beanstandungen haften, erfüllen jedoch nicht die strengen Anforderungen, die an therapeutische Systeme gestellt werden müssen. Definitionsgemäß enthält ein transdermales therapeutisches System einen oder mehrere Arzneistoffe, die in vorausbestimmter Rate kontinuierlich über einen festgesetzten Zeitraum an einen festgelegten Anwendungsort abgegeben werden sollen ("Heilmann, Klaus: Therapeutische Systeme - Konzept und Realisation programmierter Arzneiverabreichung", 4. Auflage, Ferdinand Enke Verlag Stuttgart, 1984, S. 26). Ohne dauerhaften Hautkontakt über den festgesetzten Zeitraum kann Physostigmin nicht kontrolliert abgegeben werden, wodurch die vorgesehene Therapie in Frage gestellt bzw. ernsthaft gefährdet wird.

Dem Fachmann ist bekannt, daß das Klebeverhalten eines TTS mit Physostigmin umso besser ist, je polarer der Weichmacher ist, der in das System eingearbeitet wurde.

Im experimentellen Teil wird nachfolgend gezeigt werden, daß - zwar mit Ausnahmen - die Klebkraft der erfindungsgemäß gefertigten TT-Systeme umso größer ist, je hydrophiler die Matrix ist. Als Maß für die Hydrophilie wurde die Hydrophilie der eingesetzten Weichmacher, nämlich von Alkoholen, herangezogen.

Ein weiteres Maß für die Hydrophilie der Weichmacher ist ihr HLB-Wert, der nach folgenden Formeln errechnet werden kann:

$$HLB = 20 \left(1 - \frac{Mo}{M}\right)$$

Mo = Molekülmasse des hydrophoben Anteils

M = Gesamtmolekülmasse

bzw. für Ester

$$HLB = 20 \left(1 - \frac{VZ}{SZ}\right)$$

VZ = Verseifungszahl des Esters

SZ = Säurezahl der abgetrennten Fett säure

(zitiert nach Voigt, Rudolf: Lehrbuch der pharmazeutischen Technologie, 3. überarbeitete Auflage, Verlag Chemie, Weinheim, New York 1979, S. 352)

Die HLB-Werte der Weichmacher sind in Tab. 5 wiedergegeben.

Da Weichmacher Physostigmin hydrolytisch spalten, ist es erforderlich, solche Matrices zu formulieren, die einerseits durch die Verwendung von speziellen polaren Weichmachern hydrophil sind, die aber andererseits durch die Bereitstellung von besonders geeigneten Polymerbestandteilen die Hydrolyse des Wirkstoffs verhindern.

Aus dem nächstliegenden Stand der Technik entsprechend der EP-A-0 377 147 bzw. der EP-A-0 376 067 sind transdermale therapeutische Systeme bekannt, mit Physostigmin als wirksame Bestandteil, welche Physostigmin oder dessen pharmazeutisch verträgliche Salze über einen Zeitraum vom 24 Stunden kontrolliert abgeben und gleichzeitig gewährleisten, daß sich Physostigmin während der Lagerung des transdermalen therapeutischen Systems nicht merklich zersetzt. Um die Stabilität von Physostigmin zu garantieren, wird der Wirkstoff in beiden transdermalen therapeutischen Systeme aus D1 und D2, aus einer lipophilen Matrix an die Haut abgegeben.

Aufgabe der Erfindung ist daher die Bereitstellung eines TTS, das Physostigmin oder eines seiner pharmazeutisch unbedenklichen Salze über einen Zeitraum von mindestens 12 Stunden kontrolliert abgibt, bei dem eine dauerhafte und vollflächige Fixierung des Pflasters auf dem menschlichen Körper gewährleistet und gleichzeitig sichergestellt ist, daß sich das Physostigmin im System nicht in einer die therapeutische Wirkung verändernden Weise zersetzt.

Diese Aufgabe wird erfindungsgemäß gelöst durch ein transdermales therapeutisches System gemäß Anspruch 1. Dabei kann die wirkstoffundurchlässige Rückschicht aus flexiblem oder nicht flexiblem Material bestehen. Materialien, die zu ihrer Herstellung verwendet werden können, sind Polymerfolien oder Metallfolien, wie Aluminiumfolie, die allein oder in Beschichtung mit einem polymeren Substrat angewandt werden. Es können auch textile Flächengebilde verwendet werden, wenn die Bestandteile des Reservoirs aufgrund ihrer physikalischen Beschaffenheit durch sie nicht hindurchtreten können. Bei einer bevorzugten Ausführungsform ist die Rückschicht ein Verbundstoff aus einer mit Aluminium bedampften Folie.

Ein Verfahren zur Herstellung des TTS ist entsprechend den Merkmalen der Ansprüche 7 und 8 vorgesehen.

Die Reservoirschicht besteht aus einer haftklebenden Polymermatrix und enthält den Wirkstoff Physostigmin, oder dessen pharmazeutische unbedenklichen Salze, den Weichmacher und fallweise erforderliche Zusatzstoffe.

Bei der Auswahl der Stoffe für den Aufbau des Reservoirs müssen folgende Bedingungen gleichzeitig erfüllt werden:

- Das vergleichsweise instabile Physostigmin darf während der Lagerung und/oder der Therapie weder hydrolytisch gespalten noch oxidativ angegriffen werden.

- Die Reservoirschicht muß gegenüber menschlicher Haut ein gutes Klebeverhalten über die gesamte vorgesehene Tragedauer, mindestens 12 Stunden, aufweisen.

Diese Bedingungen werden in überraschender Weise durch die Kombination der erfindungsgemäß beschriebenen Grundpolymere und Weichmacher erfüllt, wobei sich die Auswahl des Grundpolymers nach den chemischen und physikalischen Eigenschaften des Physostigmins richtet sowie die Forderung zu erfüllen hat, daß die Matrix hydrophil genug ist, um die Klebkraft beim Tragen auf der Haut zu garantieren.

Dabei wird von der Erkenntnis ausgegangen, daß nicht die Polymeren, sondern die Weichmacher Physostigmin hydrolytisch spalten. Deshalb dürfen die Weichmacher eine gewisse Hydrophilie nicht überschreiten.

Lösungsmittel wie beispielsweise Methanol, Ethanol und Isopropanol, die keine Weichmacher im Sinne der Erfindung sind, liegen mit ihren HLB-Werten zwar innerhalb der erfindungsgemäßen Grenzen, verursachen jedoch eine Zersetzung von Physostigmin. Sie dürfen daher in der Reservoirschicht der Matrix nicht verwendet werden.

Alkohole mit extrem niedrigen HLB-Werten, z.B. Heptadecanol, Nonadecanol und Eicosanol, können zwar Physostigmin nicht spalten, sie bewirken aber ein schlechteres Klebeverhalten des Systems, und stellen daher gegenüber den Triglyceriden der mittelkettigen Fettsäuren keinen Fortschritt dar.

Die für den Wirkstofffluss durch die Haut erforderliche Hydrophilie der Matrix muß infolgedessen durch Polymere mit

EP 0 667 774 B1

polaren funktionellen Gruppen wie Hydroxyl-, Carbonyl-, Carboxyl- oder Aminogruppen verwirklicht werden. Als Polymere kommen beispielsweise Polyacrylate ode Polymethacrylate mit den oben genannten polaren funktionellen Gruppen in Frage.

Ohne die Erfindung einzuschränken, werden als Polymere auf Acrylat-Basis Acrylatcopolymere aus 2-Ethylhexylacrylat, Vinylacetat und Acrylsäure mit bzw. ohne Titan- oder Aluminiumchelatester bevorzugt. Als Methacrylate werden Copolymere auf Basis von Dimethylaminoethylmethacrylaten und neutralen Methacrylsäureestern bevorzugt.

Beispiele für erfindungsgemäße Weichmacher sind höhere Alkohole, da sie einerseits hydrophil genug sind, um mit den beschriebenen Polymeren die geforderte "hydrophile Matrix" zu bilden, andererseits aber nicht polar genug sind, um Physostigmin im System via Alkoholyse zu spalten. Besonders bewährt haben sich geradkettige oder verzweigte, gesättigte oder ungesättigte Alkolhole mit 6 bis 20 Kohlenstoffatomen.

Die Art der möglichen übrigen Zusätze hängt vom eingesetzten Polymer, Weichmacher und dem Wirkstoff ab: Nach ihrer Funktion lassen sie sich einteilen in Klebrigmacher, Stabilisatoren, Trägerstoffe, diffusions- und penetrationsregulierende Zusätze oder Füllstoffe. Ohne die Erfindung einzuschränken, seien als Füllstoffe Polymere, ausgewählt aus den Gruppen der Polyvinylpyrrolidone erwähnt. Andere hierfür in Frage kommende physiologisch unbedenkliche Substanzen sind dem Fachmann bekannt.

Die ablösbare Schutzschicht, die mit der Reservoirschicht in Berührung steht und vor der Anwendung entfernt wird, besteht beispielsweise aus denselben Materialien, wie sie zur Herstellung der Rückschicht benutzt werden, vorausgesetzt, daß sie ablösbar gemacht werden, beispielsweise durch eine Siliconbehandlung.

Andere ablösbare Schutzschichten sind z. B. Polytetrafluorethylen, behandeltes Papier, Cellophan, Polyvinylchlorid u. ä. Wird das erfindungsgemäße Laminat vor Aufbringen der Schutzschicht in therapiegerechte Formate (Pflaster) aufgeteilt, so können die Schutzschichtformate ein überstehendes Ende aufweisen, mit dessen Hilfe sie leichter von dem Pflaster abgezogen werden können.

Nachweis der Stabilitätsbedingungen von Physostigmin:

Als Weichmacher werden im Vergleichsbeispiel 1 Triglyceride mittelkettiger Fettsäuren, DAB 9 (Neutralöl), HLB-Wert 1 verwendet, da dieser Weichmacher nach der Lehre von DE 38 43 239 die Stabilität von Physostigmin garantiert. Dies kann experimentell bestätigt werden. In den anderen Beispielen finden Weichmacher, ausgewählt aus der Verbindungsklasse Alkohole (verzweigt oder geradkettig, gesättigt oder ungesättigt) mit 6 bis 20 Kohlenstoffatomen Verwendung. Überraschenderweise gelingt auch hier der experimentell Stabilitätsbeweis des Physostigmin im TTS obwohl in einem Kompatibilitätstest Physostigmin von diesen Verbindungen hydrolytisch gespalten wird. Hierbei wurden jeweils etwa 50 mg Physostigminbase und etwa 100 mg Alkohol in einem verschlossenen HPLC-Gefäß drei Wochen lang unter Licht- und Luftausschluß bei 40 °C gelagert.

Danach wurden die Proben jeweils mit 1 ml Chloroform versetzt und die Färbung beurteilt. Anschließend wurden die Proben dünnschicht-chromatographisch untersucht.

Die DC-Bedingungen sind wie folgt:

Absorbens: Kieselgel DC-Glasplatte 60 F 254
Fließmittel : Chloroform : Aceton : Diethylamin 5 : 4 : 1
Kammersättigung: 2 h
Laufzeit: 35 min
Auftragsmenge: 5 μl
Detektion:

    1. UV-Licht 254 nm
    2. Jodkammer

Die Ergebnisse sind in der folgenden Tabelle 1 wiedergegeben.

4

Tabelle 1

| 50 mg Physostigmin + 100 mg "Weichmacher" | Farbe | DC-Auswertung: Abbauprodukt nachweisbar |
|---|---|---|
| 1. Triglyceride mittelkettig Fettsäuren | farblos | - |
| 2. Methanol | dunkelrot | ca. 20 % |
| 3. Ethanol | dunkelrot | ca. 15 % |
| 4. Isopropanol | dunkelrot - rot | ca. 15 % |
| 5. 1-Hexanol | dunkelrot - rot | + |
| 6. 1-Heptanol | rot | + |
| 7. 1-Octanol | hellrot | + |
| 8. 1-Nonanol | hellrot | + |
| 9. 1-Decanol | hellrot | + |
| 10. 1-Undecanol | hellrot | - |
| 11. 1-Dodecanol | hellrot | + |
| 12. 1-Tetradecanol | farblos - hellrot | in Spuren |
| 13. 1-Pentadecanol | farblos - hellrot | in Spuren |
| 14. 1-Heptadecanol | farblos | in Spuren |
| 15. 1-Nonadecanol | farblos | - |
| 16. 1-Eicosanol | farblos | - |
| 17. 2-Octyldodecanol (1) | farblos | - |
| 18. Oleylalkohol | gelb | - |

Beurteilung der Farben:

Da die Zersetzungsprodukte des Physostigmins gefärbt sind, können nur die Lösungen als stabil betrachtet werden, die farblos bleiben (Nr. 1 und 14 - 17). Gleichzeitig läßt die Intensität der Verfärbung Rückschlüsse auf den Grad der Zersetzung zu.

Die TTS wurden nach folgendem Schema hergestellt:

Das erfindungsgemäße transdermale therapeutische System wird vorzugsweise hergestellt, indem der Wirkstoff zusammen mit den Bestandteilen der haftklebenden Reservoirschicht gegebenenfalls in Lösung homogen vermischt und auf die wirkstoffundurchlässige Rückschicht aufgestrichen wird, worauf gegebenenfalls das Lösemittel oder die Lösemittel entfernt wird/werden. Anschließend wird die Klebeschicht mit einer entsprechenden Schutzschicht versehen.

Die Erfindung wird durch die folgenden weiteren Beispiele erläutert:

Beispiel 1 (Vergleichsbeispiel):

238 g eines selbstvernetzenden sauren (Säurezahl der getrockneten Masse ca. 40) Acrylatcopolymeres aus 2-Ethylhexylacrylat, Vinylacetat und Acrylsäure (47,85%ig in einem Lösungsmittelgemisch aus Isopropanol, Ethylacetat, Heptan, Toluol und Acetylaceton 26 : 37 : 32 : 4 : 1), 50 g Triglyceride der Capryl/Caprinsäuren, 16 g Physostigminbase und 20 g Ethylacetat werden unter Rühren gemischt.

Anschließend streut man unter Rühren 20 g eines kationischen Copolymerisats auf Basis von Dimethylaminoethylmethacrylat und neutralen Methacrylsäureestern ein. Unter Lichtausschluß rührt man bei Raumtemperatur 8 Stunden lang bis zur vollständigen Auflösung und streicht die erhaltene Lösung mit einem 250 $\mu$m Rakel auf eine aluminisierte und silikonisierte Polyethylen-Folie.

Nachdem das Lösemittel durch 20-minütiges Trocknen bei 60 °C entfernt wurde, deckt man den Klebefilm mit einer Polyester-Folie 15 $\mu$m ab. Mit geeigneten Schneidewerkzeugen stanzt man eine Fläche von 10 cm$^2$ aus und entfernt die gitterförmigen Ränder.

Die Stabilität des Wirkstoffs im System wurde durch Gehaltsbestimmungen direkt nach der Fertigung bzw. nach dreimonatiger Lagerung aufgezeigt. Dabei konnten weder die aus der Literatur bekannten Abbauprodukte Eserolin und Rubreserin noch andere bisher nicht beschriebene Abbauprodukte festgestellt werden. Der Gehalt an Physostigmin entsprach dem theoretischen Wert. Dazu wurde folgende Methode verwandt:

Probenvorbereitung:

1 Pflaster mit Abdeckfolie wird mittels Schere geviertelt, die Abdeckfolie entfernt und zusammen mit den Pflasterteilen in einem verschließbaren, lichtgeschützten Glasgefäß mit 50,0 ml Tetrahydrofuran (p. a.) mindestens 2 Stunden geschüttelt, 10 min ultrabeschallt und anschließend zentrifugiert. Verdünnung für HPLC mit Methanol und nochmalige Zentrifugation. Anschließend wird der Gehalt des Physostigmins im Zentrifugat per HPLC bestimmt.

Beispiel 1 besitzt durch die Verwendung von Triglyceriden - eine lipophile Matrix. Es wurde gewählt, um einen Standard zu erhalten, an dessen Stabilität die Stabilität der Beispiele 2 bis 17 gemessen werden kann.

Die Beispiele 2 - 17 wurden nach dem gleichen Schema wie Beispiel 1 gefertigt. Statt Neutralöl DAB 9 wurden höhere Alkohole mit sechs bis zwanzig Kohlenwasserstoffatomen als Weichmacher verwendet. Beispiele 15 und 16 sind keine Beispiele gemäß Erfindung. Beim Einsatz dieser Alkohole kommt es zur Zersetzung des Physostigmins.

Die qualitativen und quantitativen Zusammensetzungen der Matrices nach Entfernen der Lösemittel können der folgenden Tabelle 2 entnommen werden. Die Muster wurden nach der Herstellung und nach Lagerung bei 25 °C bzw. 40 °C nach der gleichen Methode wie Beispiel 1 untersucht. Eine Zersetzung des Wirkstoffs konnte - außer bei den Beispielen 15 und 16, die nicht der Erfindung entsprechen - nicht festgestellt werden.

Tabelle 2

| Beispiel | saures Polyacrylat | bas. Methacrylat | Physostigmin | Weichmacher |
|---|---|---|---|---|
| | | Zusammensetzung der Matrices | | |
| 2 | 67% | 10 % | 8 % | 15 % 1-Hexanol |
| 3 | 67% | 10 % | 8 % | 15 % 1-Heptanol |
| 4 | 67 % | 10 % | 8 % | 15 % 1-Octanol |
| 5 | 67 % | 10 % | 8 % | 15 % 1-Decanol |
| 6 | 67 % | 10 % | 8 % | 15 % 1-Nonanol |
| 7 | 67 % | 10 % | 8 % | 15 % 1-Undecanol |
| 8 | 77 % | 10 % | 8 % | 5 % 1-Dodecanol |
| 9 | 67 % | 10 % | 8 % | 15 % 1-Dodecanol |
| 10 | 62 % | 10 % | 8 % | 20 % 1-Dodecanol |
| 11 | 67 % | 10 % | 8 % | 15 % 1-Tetradecanol |
| 12 | 67 % | 10 % | 8 % | 15 % 1-Pentadecanol |
| 13 | 67 % | 10 % | 8 % | 15 % 1-Heptadecanol |
| 14 | 72 % | 10 % | 8 % | 10 % 1-Dodecanol |
| 15 | 67 % | 10 % | 8 % | 15 % Propandiol |
| 16 | 67 % | 10 % | 8 % | 15 % Glycerin |
| 17 | 57 % | 10 % | 8 % | 25 % 1-Octyldodecanol (1) |
| 18 | 67 % | 10 % | 8 % | 15 % 1-Oleylalkohol |

saures Polymer: Acrylatcopolymer aus 2-Ethylhexylacrylat, Vinylacetat und Acrylsäure (S.Z. des getrockneten Haftklebers: ca. 40)
basisches Methacrylat: kationisches Copolymerisat auf Basis von Dimethylaminoethylmethacrylat und neutralen Methacrylsäureestern (KOH-Zahl: ca. 180)

1-Nonadecanol und 1-Eicosanol konnten unter diesen Bedingungen nicht eingearbeitet werden.

Die Klebkräfte wurden experimentell in Anlehnung an die Prüfvorschrift A.F.E.R.A 4001 P 11 bestimmt. Als Klebkraft wird hierbei die gemessene Kraft verstanden, die erforderlich ist, um einen Prüfling mit der Fläche von 16 cm$^2$ unter einem 90° Winkel von einer Stahlplatte abzuschälen. Die angegebenen Werte sind jeweils Mittelwerte aus einer Bestimmung mit jeweils fünf Prüflingen. Dem Fachmann ist dabei bekannt, daß in den meisten Fällen dann ein hohes Haftvermögen auf menschlicher Haut beobachtet wird, wenn TTS nach A.F.E.R.A. ein gutes Klebeverhalten aufweisen.

Tab. 3

| Klebkraft der Systeme in Abhängigkeit der Kettenlänge der als Weichmacher eingesetzten Alkohole. | | |
|---|---|---|
| Beispiel | Weichmacher | Klebkraft [N] |
| 1 | 25 % Triglyceride mittelkettiger Fettsäuren DAB 9 | 3,01 |
| 2 | 15 % 1-Hexanol | 9,89 |
| 3 | 15 % 1-Heptanol | 9,73 |
| 4 | 15 % 1-Octanol | 5,70 |
| 5 | 15 % 1-Nonanol | 9,35 |
| 6 | 15 % 1-Decanol | 6,16 |
| 7 | 15 % 1-Undecanol | 5,13 |
| 9 | 15 % 1-Dodecanol | 5,53 |
| 11 | 15 % 1-Tetradecanol | 2,52 |
| 12 | 15 % 1-Pentadecanol | 1,03 |
| 13 | 15 % 1-Heptadecanol | 0,42 |

Aus Tab. 3 ist ersichtlich, daß lediglich die Systeme 11 - 13 eine geringere Klebkraft aufweisen als Beispiel 1. Ferner ist ersichtlich, daß mit zunehmender Kettenlänge des als Weichmacher eingesetzten Alkohols die Klebkraft sinkt (Ausnahmen sind lediglich Beispiel 3 und 7).

Tab. 4

| Klebkraft in Abhängigkeit vom Anteil an saurem Polyacrylat. | | | |
|---|---|---|---|
| Beispiel | 1-Dodecanol | Klebkraft [N] | saures Polyacrylat |
| 8 | 5 % | 7,65 | 77 % |
| 14 | 10 % | 6,34 | 72 % |
| 9 | 15 % | 5,53 | 67 % |
| 10 | 20 % | 4,87 | 62 % |

Man erkennt, daß die Klebkraft mit steigendem Anteil an saurem Polyacrylat zunimmt.

Tabelle 4a

| Beispiel | Weichmacher | Klebkraft [N] |
|---|---|---|
| 15 | 15 % Propandiol (1,2) | 13,27 |
| 16 | 15 % Glycerin | 12,74 |

**Tabelle 5**     HLB-Werte der Weichmacher

| | M | Mo | HLB = 20 $\left(1 - \dfrac{Mo}{M}\right)$ |
|---|---|---|---|
| Triglyceride mittel-kettiger Fettsäuren DAB 9 | ca. 504 | M-6x16=408 | 1,0* |
| Methanol | 32,04 | 15,03 | 10,6 |
| Ethanol | 46,07 | 29,06 | 7,4 |
| Isopropanol | 60,10 | 43,0 | 5,7 |
| Hexanol | 102,18 | 85,17 | 3,3 |
| Heptanol | 116,20 | 99,19 | 2,9 |
| Octanol | 130,22 | 113,21 | 2,6 |
| Nonanol | 144,24 | 127,23 | 2,4 |

| | | | |
|---|---|---|---|
| Decanol | 158,26 | 141,25 | 2,2 |
| Undecanol | 172,28 | 155,27 | 2,0 |
| Dodecanol | 186,3 | 169,29 | 1,8 |
| Tetradecanol | 214,34 | 197,33 | 1,6 |
| Pentadecanol | 228,36 | 211,35 | 1,5 |
| Heptadecanol | 256,47 | 239,46 | 1,3 |
| Nonadecanol | 284,73 | 267,25 | 1,2 |
| Eicosanol | 298,55 | 281,54 | 1,1 |
| 2-Octyldodecanol | 298,55 | 281,54 | 1,1 |
| Oleylalkohol | 268,47 | 251,46 | 1,3 |
| Glycerin | 92,10 | 41,08 | 11,1 |
| Propandiol | 76,10 | 42,09 | 8,9 |

\* Angabe nach DAB 9, Kommentar S 3399

**Patentansprüche**

1. Transdermales therapeutisches System zur Verabreichung von Physostigmin an die Haut, bestehend aus einer wirkstoffundurchlässigen Rückschicht, einer haftklebenden Reservoirschicht, die 40 bis 90 Gew.-% Polymermaterial und 0,1 bis 20 Gew.-% Physostigminbase oder eines ihrer pharmazeutisch unbedenklichen Salze enthält, sowie aus einer die Reservoirschicht abdeckenden wieder ablösbaren Schutzschicht, <u>dadurch gekennzeichnet,</u> daß die Reservoirschicht Polymermaterial auf Acrylat- und/oder Methacrylatbasis und 0,1 bis 40 Gew.% eines hydroxylgruppenhaltigen Weichmachers mit einem HLB-Wert zwischen 1,1 und 12,0 enthält, aüsgenommen eine Zersetzüng von Physostigmin bewirkende Verbindungen wie Methanol, Ethanol, Isopropanol, Propandiol oder Glycerin.

2. Transdermales therapeutisches System nach Anspruch 1, <u>dadurch gekennzeichnet,</u> daß die Reservoirschicht als Polymermaterial Acrylatcopolymere aus 2-Ethylhexylacrylat, Vinylacetat und Acrylsäure enthält.

3. Transdermales therapeutisches System nach Anspruch 1, <u>dadurch gekennzeichnet,</u> daß das Polymermaterial auf Basis von Methacrylaten ein Copolymer auf Basis von Dimethylaminoethylmethacrylat und neutralen Methacrylsäureestern enthält.

4. Transdermales therapeutisches System nach Anspruch 1, <u>dadurch gekennzeichnet,</u> daß der Weichmacher 1-Dodecanol ist.

5. Transdermales therapeutisches System nach Anspruch 1, <u>dadurch gekennzeichnet,</u> daß der Weichmacher 2-Octyldodecanol ist.

6. Transdermales therapeutisches System nach Anspruch 1, <u>dadurch gekennzeichnet,</u> daß der Weichmacher Oleyl-alkohol ist.

7. Verfahren zur Herstellung des transdermalen therapeutischen Systems nach den Ansprüchen 1 bis 6, <u>dadurch gekennzeichnet,</u> daß auf einer undurchlässigen Rückschicht eine haftklebende Reservoirschicht aufgebracht wird, wobei diese 40 bis 90 Gew.-% haftklebendes Polymermaterial und 0,1 bis 20 Gew.-% Physostigminbase oder eines ihrer pharmazeutisch unbedenklichen Salze als Wirkstoff enthält, und diese mit einer wieder ablösbaren Schutzschicht abgedeckt wird, wobei für die Reservoirschicht eine homogene Mischung eines Polymermaterials auf Acrylat- und/oder Methacrylatbasis mit 0,1 bis 40 Gew.-% eines hydroxylgruppennaltigen Weichmachers mit einem HLB-Wert zwischen 1,1 und 12,0 verwendet wird, aüsgenommen eine Zersetzüng von Physostigmin bewirkende Verbindungen wie Methanol, Ethanol, Isopropanol, Propandiol oder Glycerin.

8. Verfahren nach Anspruch 7, <u>dadurch gekennzeichnet,</u> daß der Wirkstoff zusammen mit den Bestandteilen der klebfähigen Reservoirschicht gegebenenfalls in Lösung homogen Vermischt und auf die wirkstoffundurchlässige Rückschicht aufgestrichen wird, worauf das Lösemittel oder die Lösemittel - soweit vorhanden - entfernt und anschließend die Klebschicht mit einer Schutzschicht abgedeckt und anschließend das Laminat durch Zerteilen zu einzelnen TTS konfektioniert und diese in eine Verpackung eingesiegelt werden.

## Claims

1. Transdermal therapeutic system for the administration of physostigmine to the skin, consisting of a backing layer impermeable to active substance, a pressure-sensitive reservoir layer containing 40 to 90 percent by weight polymeric material and 0.1 to 20 percent by weight physostigmine base or one of its pharmaceutically acceptable salts, and of a removable protective layer covering the reservoir layer, <u>characterized in that</u> the reservoir layer contains polymeric material based on acrylates and/or methacrylates and 0.1 to 40 percent by weight of a hydroxyl group-containing softener having an HLB value between 1.1 and 12.0 with the exception of compounds causing the disintegration of physostigmine, such as methanol, ethanol, isopropanol, propanediol or glycerol.

2. Transdermal therapeutic system according to claim 1, <u>characterized in that</u> the reservoir layer contains as polymeric material acrylate copolymers of 2-ethylhexyl acrylate, vinyl acetate and acrylic acid.

3. Transdermal therapeutic system according to claim 1, <u>characterized in that</u> the polymeric material based on methacrylates contains a copolymer based on dimethylaminoethyl methacrylate and neutral methacrylic esters.

4. Transdermal therapeutic system according to claim 1, <u>characterized in that</u> the softener is 1-dodecanol.

5. Transdermal therapeutic system according to claim 1, characterized in that the softener is 2-octyl dodecanol.

6. Transdermal therapeutic system according to claim 1, <u>characterized in that</u> the softener is oleyl alcohol.

7. Process for the production of a transdermal therapeutic system according to claims 1 to 6, <u>characterized in that</u> to an impermeable backing layer there is applied a pressure-sensitive reservoir layer, whereby said pressure-sensitive reservoir layer contains 40 to 90 percent by weight pressure-sensitive polymeric material and 0.1 to 20 percent by weight physostigmine base or one of the pharmaceutically acceptable salts thereof as active substance, and that said layer is covered with a removable protective layer, whereby for the reservoir layer there is used a homogeneous mixture comprising a polymeric material based on acrylates and/or methacrylates, and 0.1 to 40 percent by weight of a hydroxyl group-containing softener having an HLB-value between 1.1 and 12.0 with the exception of compounds causing the disintegration of physostigmine, such as methanol, ethanol, isopropanol, propanediol or glycerol.

8. Process according to claim 7, <u>characterized in that</u> the active substance is homogeneously mixed together with the components of the adhesive reservoir layer, optionally in solution, and spread onto the backing layer impermeable to active substance, whereafter the solvent or solvents - where present - are removed and, subsequently, the adhesive layer is covered with a protective layer, whereafter the laminate is manufactured into individual TTS by separating, which TTS are sealed into a packaging.

## Revendications

1. Système thérapeutique transdermique pour l'administration de physostigmine à la peau, qui se compose d'une couche dorsale imperméable au principe actif, d'une couche formant réservoir autocollante, qui contient 40 à 90% en poids de matière polymérique et 0,1 à 20% en poids de physostigmine-base ou d'un de ses sels pharmaceutiquement acceptables, ainsi que d'une couche protectrice amovible recouvrant la couche formant réservoir, caractérisé en ce que la couche formant réservoir contient de la matière polymérique à base d'acrylate et/ou de méthacrylate et 0,1 à 40% en poids, d'un plastifiant contenant des radicaux hydroxyle, d'un indice HLB compris entre 1,1 et 12,0, à l'exclusion de composés provoquant la décomposition de la physostigmine, comme le méthanol, l'éthanol, l'isopropanol, le propanediol, ou la glycérine.

2. Système thérapeutique transdermique suivant la revendication 1, caractérisé en ce que la couche formant réservoir contient, à titre de matière polymérique, des copolymères d'acrylate constitués d'acrylate de 2-éthylhexyle, d'acétate de vinyle et d'acide acrylique.

3. Système thérapeutique transdermique suivant la revendication 1, caractérisé en ce que la matière polymérique à base de méthacrylates contient un copolymère à base de méthacrylate de diméthylaminoéthyle et d'esters de l'acide méthacrylique neutres.

4. Système thérapeutique transdermique suivant la revendication 1, caractérisé en ce que le plastifiant est le 1-dodécanol.

5. Système thérapeutique transdermique suivant la revendication 1, caractérisé en ce que le plastifiant est le 2-octyldodécanol.

6. Système thérapeutique transdermique suivant la revendication 1, caractérisé en ce que le plastifiant est l'alcool oléylique.

7. Procédé de préparation du système thérapeutique transdermique suivant les revendications 1 à 6, caractérisé en ce que l'on applique sur la couche dorsale imperméable, une couche formant réservoir autocollante, où cette dernière contient de 40 à 90% en poids d'une matière polymérique autocollante et 0,1 à 20% en poids de physostigmine-base ou d'un de ses sels pharmaceutiquement acceptables, à titre de principe actif et cette couche formant réservoir est recouverte d'une couche de protection amovible, où on utilise pour la couche formant réservoir un mélange homogène d'une matière polymérique à base d'acrylate et/ou de méthacrylate et de 0,1 à 40% en poids d'un plastifiant contenant des radicaux hydroxyle et d'un indice HLB compris entre 1,1 et 12,0, à l'exclusion de composés provoquant la décomposition de la physostigmine, comme le méthanol, l'éthanol, l'isopropanol, le propanediol, ou la glycérine.

8. Procédé suivant la revendication 7, caractérisé en ce que l'on mélange de façon homogène le principe actif aux constituants de la couche formant réservoir collante éventuellement en solution et on l'étale sur la couche dorsale imperméable au principe actif, puis on élimine le solvant ou les solvants - pour autant qu'ils fussent présents - et on recouvre ensuite la couche de colle d'une couche protectrice et on confectionne ensuite le lamifié ou stratifié par subdivision en un STT individuel et on scelle ou enferme hermétiquement celui-ci dans un emballage.